# EUROPEAN PATENT SPECIFICATION

(11) **EP 1 227 686 B1**
(45) Date of publication and mention of the grant of the patent: **22.06.2005**
(21) Application number: 00956852.8
(22) Date of filing: 31.08.2000
(51) Int. Cl.: A61B 1/04, A61B 5/00, H04N 9/04

(54) **FEEBLE LIGHT COLOR IMAGING DEVICE**
FARBBILDAUFNAHMEGERÄT FÜR SCHWACHES LICHT
DISPOSITIF D'IMAGERIE EN COULEUR A FAIBLE LUMIERE

(30) Priority: 01.09.1999 JP 24764999; 16.09.1999 JP 26230899
(43) Date of publication of application: 31.07.2002
(73) Proprietor: Hamamatsu Photonics K.K., Hamamatsu-shi, Shizuoka-ken 435-8558 (JP)
(72) Inventor: TAKEYAMA, Kaneyoshi, Hamamatsu Photonics K.K., Hamamatsu-shi, Shizuoka 435-8558 (JP)
(74) Representative: Musker, David Charles
(86) International application number: PCT/JP2000/005929
(87) International publication number: WO 2001/017272

(56) References cited:
- JP-A- 7 155 291
- JP-A- 8 223 588
- JP-A- 10 151 104
- JP-A- 59 040 830
- US-A- 5 001 556
- US-A- 5 092 331
- US-A- 5 749 830
- US-A- 5 769 792
- US-A- 5 827 190

## Description

### TECHNICAL FIELD

The present invention relates to a weak light color imaging device for capturing images of weak light from a subject, and more particularly, to a weak light color imaging device characterized in that images are captured by emphasizing fluorescent light from the subject.

### BACKGROUND ART

In recent years, much attention has been paid to the new cancer diagnosis methods and therapy methods of photodynamic diagnosis (PDD) of cancer cells which involves injecting the patient with a photosensitive material which accumulates to a higher degree in cancer cells than normal cells, and then studying the fluorescence generation characteristics when the photosensitive material is excited by light of a particular wavelength, and photodynamic therapy (PDT) which utilizes the fact that cancer cells are broken down by generation of singlet oxygen when the photosensitive material is excited by light of a particular wavelength.

Hematoporphyrin derivative (HpD) is often used as the photosensitive material in PDD and PDT. To describe PDD using HpD, the HpD emits fluorescence having peaks at wavelengths of 630 nm and 690 nm, when irradiated by excitation light of wavelength 405 nm. Therefore, if excitation light of wavelength 405 nm is irradiated onto cancer cells where HpD has accumulated, and the vicinity thereof, then fluorescence having peaks at wavelengths 630 nm and 690 nm will occur in the cancer cells where a large amount of HpD has accumulated, whereas the normal cells where hardly any HpD has accumulated will generate no fluorescence. PDD involves detecting this fluorescence in order to diagnose the location of cancer cells.

US 5, 749, 830 describes a fluorescent endoscope apparatus having: an endoscope that irradiates a subject portion to be observed with light transmitted through a light transmission device to obtain an object image of the subject portion to be observed; a normal observation light generating device for emitting normal light for performing normal light endoscope observation; a fluorescent observation light generating device for emitting excitation light for performing fluorescent light observation; an introduced-light switching device for selectively introducing, to the light transmission device of the endoscope, normal light emitted by the normal observation light generating device or excitation light emitted by the fluorescent observation light generating device; and an image sensing device for capturing a normal light image of the subject portion to be observed that can be obtained by irradiating the subject portion to be observed with normal light or a fluorescent image that can be obtained due to irradiation with excitation light, the image sensing device being included or connected to the endoscope.

### DISCLOSURE OF THE INVENTION

However, the fluorescence from the cancer cells is very weak, and in the early stages of cancer, only extremely weak fluorescence is generated. Therefore, a light amplifying tube known as an 'image intensifier' (I.I.) is used in PDD to amplify the light intensity when capturing fluorescent images. However, if a light amplifying tube is used, then wavelength information is lost, and hence in the image of the light intensity obtained from the region of cancer cells, it will be difficult to tell which portion of the image shows fluorescence having peaks at wavelengths 630 nm and 690 nm emitted from the cancer cells, and to identify whether the image shows reflected light or fluorescence of other wavelengths emitted from other materials. Consequently, there has been increasing demand for the development of a device capable of capturing images of the region of cancer cells, in color and in moving images, so that the state of the peripheral region of cancer cells can be viewed simultaneously with the fluorescence from the cancer cells.

In order to obtain color images, a color camera should be used, but it is not possible to use a standard color camera in order to capture images of weak light, such as the fluorescence from cancer cells. A conventional weak light color imaging device is either a cold CCD color camera, or an astronomical 3 CCD color camera, or the like, but a cold CCD color camera can only produce stationary images, whilst an astronomical 3 CCD color camera is a large and expensive apparatus, and therefore, if a standard device is used, it will be extremely bulky and costly.

A further prior art technique is a method wherein images taken when illumination light is irradiated onto the subject, and images wherein excitation light is irradiated onto the subject are captured separately, the images of the peripheral region of the subject and the images of fluorescence from the cancer cells are formed into a single image by superimposition, and this single image is repeatedly captured and displayed, whereby the subject can be observed by means of moving images. However, since the illumination light and the excitation light are not irradiated onto the subject at precisely the same time when it is imaged, a problem arises in that it is not possible to observe in real time, for example, the extinguishing of fluorescence due to the destruction of cancer cells in PDT.

Therefore, it is an object of the present invention to provide a weak light color imaging device capable of capturing clear images of the fluorescence from a photosensitive material accumulated in cancer cells in particular, which can be constructed inexpensively by means of a single detector, and which is able to provide moving images in real time.

In order to achieve the aforementioned object, the present invention provides a weak light color imaging device according to claim 1.

According to the present invention, the light intensity from the subject is amplified and images are captured in real time. Furthermore, the images of the red, green and blue wavelength components of the weak light are captured respectively in synchronism with a television vertical synchronization signal, and superimposing these images, a single color image is obtained. Moreover, by adjusting the quantity of the illumination light, it is possible to capture images without the weak fluorescence generated by the subject due to the exciting means being swamped by the illumination light, and hence the appearance of the peripheral region of the subject can be captured simultaneously in a moving color image.

Desirably, the output varying means varies the output of the illuminating means in such a manner that the intensity of the light from the illuminating means incident on the imaging means due to reflection or scattering by the subject is equal to, or equal to or less than, the light intensity of the fluorescence incident on the imaging means from the subject.

Moreover, it is also possible for the filtering means of the weak light color imaging device according to the present invention to transmit a greater quantity of the red wavelength component of the weak light from the subject than the blue and green wavelength components. Thereby, since images containing a large quantity of red wavelength component are captured, it is possible to obtain color images wherein the fluorescence from cancer cells which is included in the red wavelength component is emphasized.

Moreover, the filtering means of the weak light color imaging device according to the present invention may transmit a greater quantity of the red wavelength component of the weak light from the subject than the blue and green wavelength components, by transmitting the red wavelength component for a longer period of time than the blue and green wavelength components. Thereby, similarly to the aforementioned aspect of the present invention, since images containing a larger red wavelength component of the respective red, green and blue wavelength components of the weak light from the subject is captured, it is possible to obtain a color image wherein the fluorescence from cancer cells which is included in the red wavelength component is emphasized.

Furthermore, the filtering means of the weak light color imaging device according to the present invention may also transmit a greater quantity of the red wavelength component of the weak light from the subject than the blue and green wavelength components, by transmitting the red wavelength component at a higher transmissivity than the green and blue wavelength components. Thereby, similarly to the aforementioned aspect of the present invention, since images containing a larger red wavelength component of the respective red, green and blue wavelength components of the weak light from the subject is captured, it is possible to obtain a color image wherein the fluorescence from cancer cells which is included in the red wavelength component is emphasized.

Moreover, the filtering means of the weak light color imaging device according to the present invention may also transmit a wavelength component in the infrared region. Thereby, even if the wavelength from the subject includes infrared wavelength components, since this is also captured, it is possible to obtain images containing infrared wavelength information. More particularly, even if the photosensitive material accumulated in the cancer cells generates fluorescence in the infrared wavelength region, it is possible to obtain images which contain this fluorescence.

### BRIEF DESCRIPTION OF THE DRAWINGS

Fig. 1 is an is an approximate diagram of a weak light color imaging device relating to a first embodiment of the present invention;
Fig. 2A and Fig. 2B is a diagram showing the structure of a variable-output white light source;
Fig. 3 is a diagram showing the fluorescence intensity of HpD at respective positions of a body containing cancer cells which has been injected with HpD;
Fig. 4 is a diagram showing wavelength transmission characteristics of a RGB filter;
Fig. 5A is a diagram showing the composition of an RGB filter divided into four regions constituting a first embodiment;
Fig. 5B is a diagram showing the composition of an RGB filter divided into three regions constituting a first embodiment;
Fig. 6 is a diagram showing amplification characteristics with respect to the wavelength of the light amplifying tube constituting a first embodiment;
Fig. 7A - Fig. 70 shows timing charts of the action of a first embodiment, in a case where the RGB filter in Fig. 5A is used;
Fig. 8A - Fig. 8M shows timing charts of the action of a first embodiment, in a case where the RGB filter in Fig. 5B is used; and
Fig. 9 is an approximate diagram of a weak light color imaging device relating to a second embodiment of the present invention.

### BEST MODE FOR CARRYING OUT THE INVENTION

Below, one embodiment of the present invention is described with reference to the accompanying drawings. In the description of the drawings, the same symbols are applied to the same constituent elements, and duplicated descriptions are omitted.

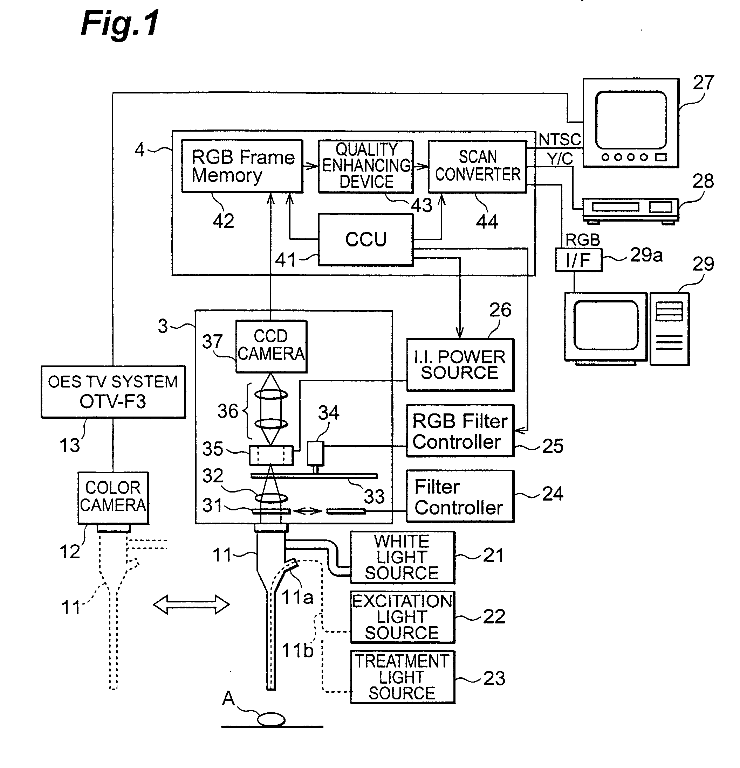

To begin, a first embodiment of the present invention will be described. Fig. 1 shows an illustrative diagram of a first embodiment. This first embodiment is a device which uses a weak light color imaging device according to the present invention in an endoscope 11. As illustrated in Fig. 1, the weak light color imaging device according to the first embodiment comprises an endoscope 11 having three types of light source 21, 22 and 23 opposing a subject A, a camera box 3 which amplifies and captures images of the weak light from the subject A guided by the endoscope 11, into respective red, green and blue wavelength components, an RGB control unit 4 for obtaining a single image by computing the image signals of the respective wavelength components thus captured, a monitor 27, VTR 28 and PC 29 for displaying and recording the captured image, and other peripheral devices.

In the first embodiment, in order to capture an image of a subject A comprising cancer cells, using a weak light color imaging device, it is necessary for the endoscope 11 to be inserted inside the patient's body and for the front tip of the endoscope 11 to be opposing the subject A. Therefore, firstly, a color camera 12 as connected to the endoscope 11, and then the position of the subject A is located. As shown in Fig. 1, the endoscope 11 having a variable-output fluorescent light source 21 (Xe lamp), and the illumination from the white light source 21 is directed by a light guide (not illustrated) to the front tip of the endoscope 11, where it is irradiated onto the interior of the body. The color camera 12 is connected via an OES TV SYSTEM 13 to a monitor 27. Thereby, an image of the interior of the body faced by the endoscope 11 is projected on the monitor 27. The operator controls the endoscope 11 appropriately so as to find the subject A by means of the images displayed on the monitor 27. When the subject is discovered, the endoscope 11 makes a one-touch connection with the connection section for the endoscope 11 on the camera box 3. Thereby, the endoscope 11 faces the subject A, and the weak light from the subject A it is facing is guided by the image guide (not illustrated) of the endoscope 11 to the camera box 3. Thereafter, the subject A is observed by means of the weak light color imaging device according to the first embodiment. Incidentally, it is supposed that hematoporphyrin derivative, which is a photosensitive material, has been injected previously into the patient's body.

Next, the composition of the endoscope 11 will be described. As shown in Fig. 1, the endoscope 11 comprises a variable-output white light source 21 which irradiates illumination light onto the subject A by means of a light guide (not illustrated) provided in the endoscope 11 as described above, an excitation light source 22 (Hg-Xe lamp) for generating characteristic HpD fluorescence by irradiating excitation light, as used in photodynamic diagnosis (PDD), and a treatment light source 23 (excimer dye laser) for destroying cancer cells by irradiating treatment light, as using in photodynamic therapy (PDT). The light from these sources is irradiated onto the subject A by being guided by fibres 11b comprising connectors inserted into an eyelet 11a attached to the endoscope 11.

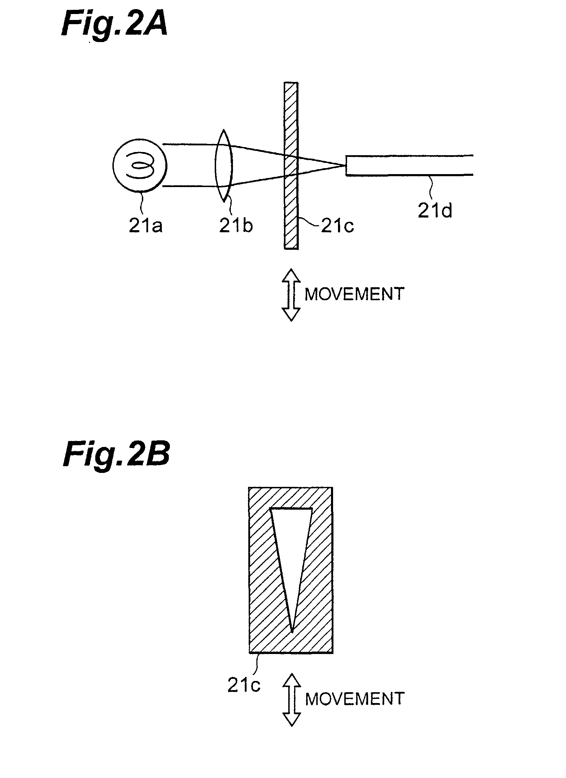

Fig. 2A and Fig. 2B show the internal structure of a variable-output white light source 21. As shown in Fig. 2A, this white light source 21 comprises an Xe lamp 21a forming the light source, a condensing lens 21b for condensing the light of the Xe lamp 21a, and a shield plate 21c for shielding the condensed light. The light transmitted by the shield plate 21c is directed via an optical fibre 21d into the endoscope 11. As shown in Fig. 2B, the shield plate 21c has an isosceles triangular shaped hole section in the vertical direction thereof, and when light from the light source 21a is incident on the shield plate 21c, the light incident on the hole section is transmitted without alteration, whilst the rest of the light is shielded by the shield plate 21c. Therefore, if the shield plate is moved upwards and downwards, then the amount of light that is able to pass the shield plate 21c changes according to the width of the isosceles triangular shaped hole section. Since the white light source 21 is constituted in this way, the output thereof can be varied.

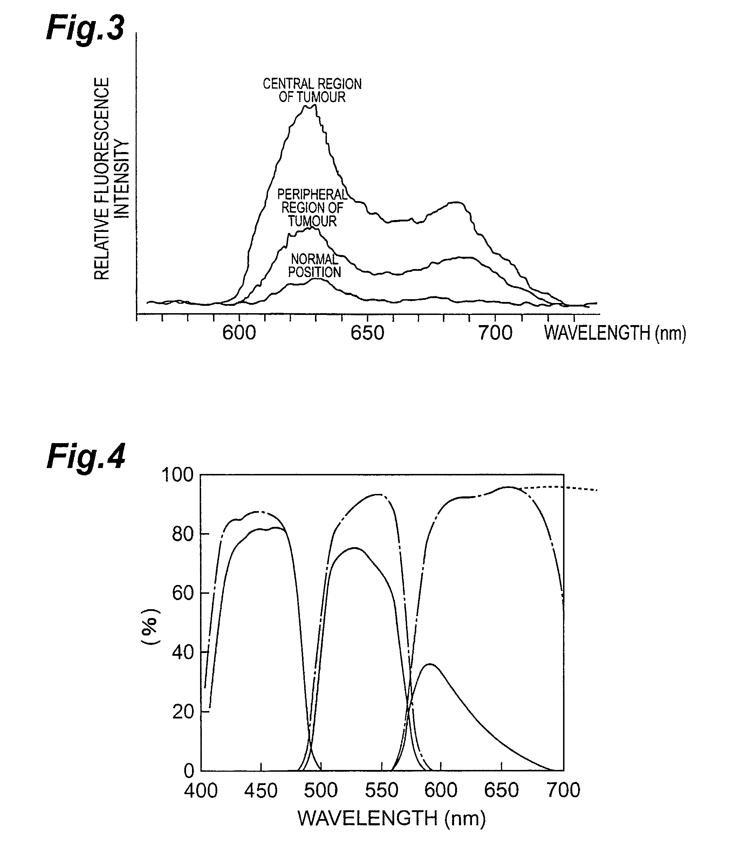

Fig. 3 shows the fluorescence spectrum of HpD in different locations when excitation light is irradiated onto a body containing cancer cells, into which HpD has been injected. As shown in this diagram, since the cancer cells (tumour) accumulate the HpD selectively, fluorescence having peaks at wavelengths 630 nm and 690 nm as characteristic of HpD is generated. However, in the peripheral region of the cancer cells, where the amount of HpD absorbed in low, the HpD fluorescence generated is of lower intensity than in the cancer cells. Since normal cells hardly accumulate HpD, there is virtually no HpD fluorescence. PDD is a diagnosis method which uses the characteristics of the HpD accumulated selectively in the cancer cells in this way to diagnose cancers by detecting fluorescence of the characteristic wavelength of HpD. In the first embodiment of the present invention, excitation light is irradiated onto the subject A by an excitation light source 22, the fluorescence of the HpD is captured as images by the weak light color imaging device, and the observer is able to diagnose cancer cells in the peripheral region of the subject A, in other words, to perform PDD, by observing the fluorescence. Moreover, if the position of the cancer calls can be confirmed, then it is also possible to perform PDT at that position by changing the excitation light source 22 for the treatment light source 23 and irradiating treatment light on to the cancer cells.

Next, the composition of a camera box 3 is described. As shown in Fig. 1, weak light from the subject A is guided by the endoscope 11 to the camera box 3. The camera box 3 comprises an excitation light excluding filter 31 for excluding the excitation light components reflected for scattered by the subject A when the excitation light source 22 is used, a coupling lens 32 for condensing the weak light from the subject A, an RGB filter 33 for respectively and separately transmitting the red, green and blue components of the condensed weak light from the subject A, a rotational motor 34 for causing the RGB filter 33 to rotate, a light amplifying tube 35 for amplifying the light transmitted by the RGB filter 33, and a CCD camera 37 for capturing images of light amplified by the light amplifying tube 35, by means of a relay lens 36.
the excitation light excluding filter 31 is connected to a filter controller 24, and when the excitation light source 22 is being used, the excitation light excluding filter 31 is inserted inbetween the endoscope 11 and coupling lens 32 and excludes the excitation light component contained in the weak light from the subject A, whereas when the excitation light source 22 is not being used, the excitation light excluding filter 31 is moved and controlled so that the weak light from the subject A guided by the endoscope 11 is conducted directly onto the coupling lens 32. Moreover, the rotational motor 34 is coupled to an RGB filter controller 25, which controls the RGB filter 33 in such a manner that it rotates in synchronism with a synchronizing signal, as described hereinafter. Furthermore, the light amplifying tube 35 is connected to a light amplifying tube power source 26, which is controlled in such a manner that a gate function of the light amplifying tube 35 is activated by switching the light amplifying tube power source 26 ON and OFF in synchronism with the synchronizing signal, as described hereinafter.

The wavelength transmission characteristics of the RGB filter 33 used in the first embodiment are now described with reference to Fig. 4. The RGB filter is constituted by filters which respectively transmit light of the three basic colors, red (R), green (G) and blue (B). Fig. 4 is a diagram showing the relationship between the wavelength of the light incident on the RGB filter and the transmissivity thereof; and it depicts respective wavelength transmission characteristics for the three filters, red, green and blue. The solid line indicate the wavelength transmission characteristics of the RGB filter when used in a standard CCD camera. It can be seen that the transmissivity increases as the wavelength shortens, from red, through green to blue. These wavelength transmission characteristics are set in order to obtain an image which corresponds to the visual receptivity of humans, in such a manner that the colors of the obtained images correspond to the colors of the images as actually seen by the human eye. The single-dotted line indicates the wavelength transmission characteristics of an RGB filter 33 having raised transmissivity of components in the longer wavelength, red region, as used in the first embodiment. Here, it can be seen that in contrast to the standard RGB filter illustrated by the solid line, the transmissivity increases as the wavelength lengthens from blue, through green to red. Therefore, the image thus obtained has stronger intensity in the longer-wavelength components, and particularly, the red components. Moreover, the characteristics indicated by the dotted line on the longer wavelength side show the wavelength transmission characteristics of an RGB filter 33 designed in such a manner that light of infrared wavelengths is also transmitted. By transmitting light of the infrared wavelengths also, information from the infrared region is included in the images captured by the CCD camera 37.

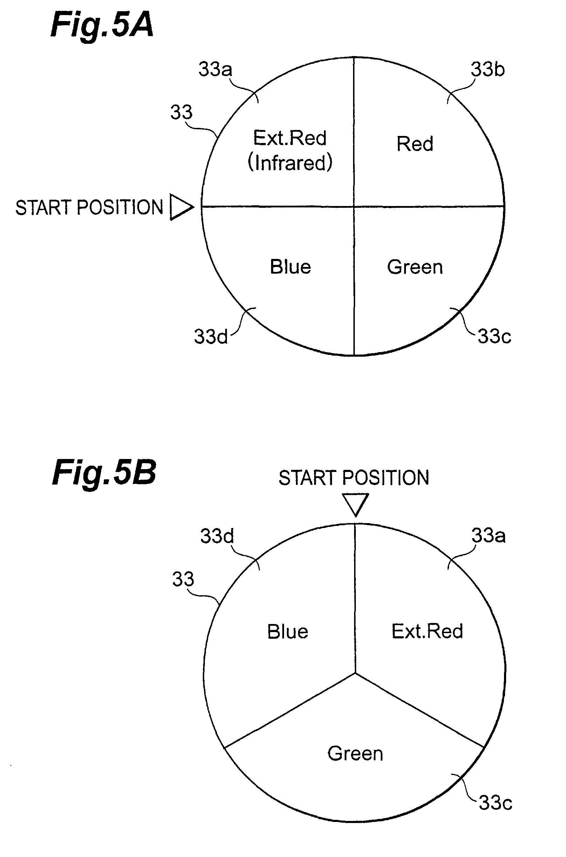

The composition of the RGB filter 33 is now described with reference to Fig. 5A and Fig. 5B. Fig. 5A shows an embodiment wherein a circular disc forming an RGB filter 33 is used in 4 separate divisions. The respective divided regions comprise an Extended Red (Ext. R) region 33a which transmits red wavelength components extending into the infrared region, having the wavelength transmission characteristics illustrated by the dotted line in Fig. 4, an R region 33b which transmits red wavelength components having the wavelength transmission characteristics illustrated by the single dotted line in Fig. 4, a G region 33c which transmits green wavelength components, and a B region 33d which transmits blue wavelength components. When the RGB filter 33 is caused to rotate as a uniform speed, and light is passed through it at a certain uniform position, the light is split in a time series fashion into separate wavelength components corresponding to the wavelength transmission characteristics of the respective regions. In the RGB filter 33 illustrated in Fig. 5A, the Ext. R region 33a and the R region 33b occupy one half of the area of the RGB filter 33, and therefore the red wavelength components are transmitted for a longer period of time than the green and blue wavelength components. Moreover, since the RGB filter 33 transmits components on the longer wavelength side shown in Fig. 4 with greater transmissivity, the longer wavelength components, and especially the red wavelength components, are transmitted in greater quantity than in the case of a normal RGB filter.

Fig. 5B illustrates an embodiment wherein the circular disc forming the RGB filter 33 is divided into three regions. The respective divided regions comprise an Extended Red (Ext. R) region 33a which transmits red wavelength components extending into the infrared region, having the wavelength transmission characteristics indicated by the dotted line in Fig. 4, a G region 33c which transmits green wavelength components, having the wavelength transmission characteristics indicated by the single-dotted line in Fig. 4, and a B region 33d which transmits blue wavelength components. With the RGB filter 33 shown in Fig. 5B, similarly to that shown in Fig. 5A, the light passing through the RGB filter 33 can be split in time series fashion into separate wavelength components corresponding to the wavelength transmission characteristics of the respective regions. Moreover, since the RGB filter 33 transmits components on the longer wavelength side shown in Fig. 4 with greater transmissivity, the longer wavelength components, and especially the red wavelength components, are transmitted in greater quantity than in the case of a normal RGB filter. The triangular symbols on Fig. 5A and Fig. 5B indicate the start position of the RGB filter 33 when it is rotated by the rotational motor 34 in synchronism with the synchronizing signal, as described hereinafter.

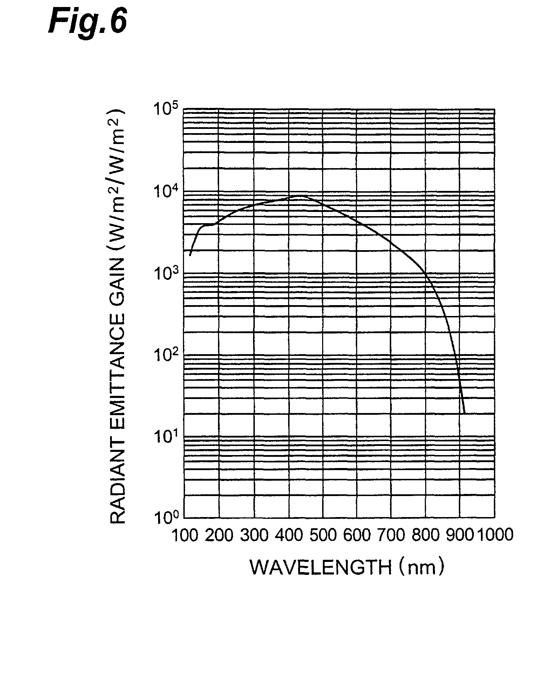

The wavelength amplification characteristics of the light amplifying tube 35 used in the first embodiment are now described with reference to Fig. 6. The light amplifying tube 35 used in the first embodiment has extended sensitivity into the longer wavelength, infrared region, and as shown in the diagram, it has satisfactory gain for light between wavelengths of 100 nm and 900 nm. Therefore, it is capable of amplifying light of various wavelength components, from infrared to blue light, transmitted by the RGB filter 33.

Next, the composition of the RGB control unit 4 will be described. As shown in Fig. 1, an image signal of various wavelength components capture by the CCD camera 37 is sent to the RGB control unit 4. The RGB control unit 4 comprises a communications control unit (CCU) 41, an RGB frame memory 42 for storing image signals form the CCD camera 37, an image quality enhancing device 43 for improving image quality by removing noise form the image signal, and a scan converter 44 for converting the scanning of the image signal which has been quality enhanced by the quality enhancing device 43, in accordance with the medium onto which the respective images are to be written, as described hereinafter.

The CCU 41 is a control unit which causes the wavelength color imaging device of the first embodiment to operate in synchronism with a 60Hz television vertical synchronizing signal (VD). The CCU 41 sends a start signal to the RGB filter controller 25, whereby the RGB filter 33 is controlled in such a manner that the start position of the RGB filter 33 as marked by the triangle symbols in Fig. 5A and Fig. 5B rotates in synchronism with a start signal. Moreover, it also sends a light amplifying tube gate signal to the light amplifying tube power source 26, causing the light amplifying tube power source 26 to switch on and off and hence realizing a gate function of the light amplifying tube 35, in such a manner that only the valid light components transmitted by the Ext. R region 33a, R region 33b, G region 33c and B region 33d shown in Fig. 5A are amplified according to the rotation of the RGB filter 33. In other words, in the boundary areas between the divided regions in Fig. 5A, it is possible that light components transmitted by both regions are mixed together, and hence the light transmitted by the boundary areas between regions is treated as an invalid component and is excluded by means of a gate function of the light amplifying tube 35, whereby only light transmitted by the central areas of each filter is taken as valid components and amplified by the light amplifying tube 35.

The CCU 41 controls the RGB frame memory 42 and the scan converter 44 in the RGB control unit 4. The RGB frame memory 42 is controlled in such a manner that it reads out the respective image signals of the extended red (red including infrared), red, green, and blue wavelength components captured in time series by the CCD camera 37, in synchronism with a CCD charge read-out signal sent by the CCU 41, and it stores the respective image signals thus read out in respective corresponding video frame memories (VFM). The scan converter 44 reads out the respective image signals of the extended red, red, green and blue wavelengths stored in the RGB frame memory 42, via the image quality enhancing device 43, and computes these signals as a single color image frame. Thereupon, it converts the scanning to image signals corresponding to the media to which the respective images are to be input, in synchronism with the (Ext. R + R + G + B) read-out signal from the CCU 41. As shown in Fig. 1, in the first embodiment, the media to which the images are input are a monitor 27, VTR 28 and PC 29. The image signal input to the monitor 27 is converted to an NTSC signal, the image signal input to the VTR 28 is converted to a Y/C signal, and the image signal input to the PC is converted to an RGB signal. Here, the PC 29 reads in the converted RGB signal by means of an interface circuit (I/F) 29a.

Next, the action of the weak light color imaging device according to the first embodiment will be described with reference to Fig. 1 and Fig. 7A - Fig. 70.

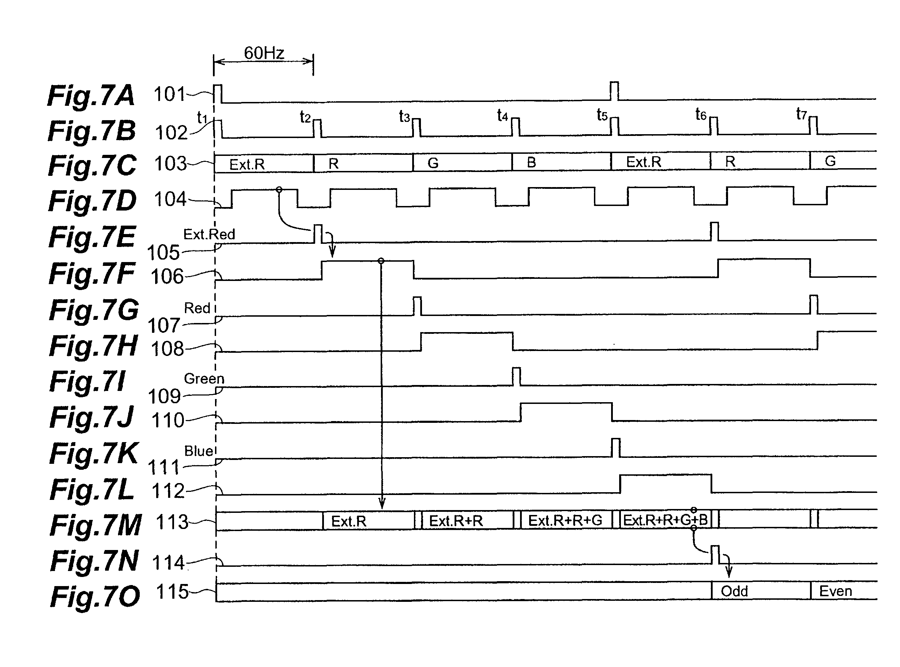

Fig. 7A - Fig. 70 are timing charts indicating the operations performed for each signal from inputting images of the various wavelength components, extended red, red, green and blue, in synchronism with a 60 Hz VD signal, through computation of the respective images, until generation of a single color image. In the diagram, chart 101 shows the start signal, chart 102 shows the video signal (VD), chart 103 shows the RGB filter signal, chart 104 shows the gate signal of the light amplifying tube 35, chart 105 shows the Ext. R charge read-out signal of the CCD, chart 106 shows the Ext. R VFM input signal, chart 107 shows the CCD Red charge read-out signal, chart 108 shows the Red VFM input signal, chart 109 shows the CCD Green charge read-out signal, chart 110 shows the Green VFM input signal, chart 111 shows the CCD Blue charge read-out signal, chart 112 shows the Blue VFM input signal, chart 113 shows the (Ext. R + R + G + B) computing signal, chart 114 shows the (Ext. R + R + G + B) read-out signal, and chart 115 shows the interlace scan converting signal.

Let it be supposed that subject A is illuminated by the white light source 21 only, and that the excitation light source 22 and the treatment light source 23 are turned off. The weak light from the subject A is conducted by the endoscope 11 to the camera box 3, where it is condensed by the coupling lens 32 (in this case, the excitation light excluding filter 31 is moved from between the endoscope 11 and the coupling lens 32 by the control of the filter controller 24). The condensed weak light is input to and filtered by the RGB filter 33. Here, a filter comprising four divisions as illustrated in Fig. 5A is used as the RGB filter 33.

At time point t₁, the start signal shown in chart 101 rises, and in synchronism with this, the RGB filter 33 is rotated starting from the start position indicated by the triangle symbol in Fig. 5A. In the period from time point t₁ to time point t₂, the weak light is transmitted by the Ext. R region 33a of the rotating RGB filter 33, as shown in chart 103. The extended red wavelength component of weak light thus transmitted is incident on the light amplifying tube 35, but due to the gate function of the light amplifying tube 35 as illustrated in chart 104, only the valid component passing through the Ext. R region 33a is amplified by the light amplifying tube 35. The light of extended red wavelength components amplified by the light amplifying tube 35 is captured by the CCD camera 37 which accumulates an electric charge corresponding to an image signal.

At time point t₂, the CCD charge read-out signal rises as shown in chart 105, whereby the electric charge corresponding to the image signal of the extended red wavelength components accumulated by the CCD camera 37 is output to the RGB frame memory 42 of the RGB control unit 3. In the time period from time point t₂ to time point t₃, as shown in chart 103, the wavelength is transmitted by the R region 33b of the rotating RGB filter 33. The R wavelength component transmitted thereby is incident on the light amplifying tube 35, but due to the gate function of the light amplifying tube 35 as illustrated in chart 104, only the valid component passing through the R region 33b is amplified by the light amplifying tube 35. The light of the red wavelength component thus amplified by the light amplifying tube 35 is captured by the CCD camera 37 and an electric charge corresponding to an image signal is accumulated. The image signal of the extended red wavelength components output to the RGB frame memory 42 is written to the Ext. R VFM of the RGB frame memory 42, as illustrated in chart 106. The characteristic noise components generated by amplification of the light in the light amplifying tube 35 are removed from the image signal of the extended red wavelength components written to the Ext. R VFM, by means of the image quality enhancing device 43. Thereupon, the image signal is input to a computing section (not illustrated) of the scan converter 44, as shown in chart 113.

At time point t₃, the CCD charge read-out signal rises, as shown in chart 107, whereby the electric charge corresponding to the image signal of the red wavelength component accumulated by the CCD camera 37 is output to the RGB frame memory 42 of the RGB control unit 3. In the time period from time point t₃ to t₄, the wavelength is transmitted by the G region 33c of the rotating RGB filter 33, as shown in chart 103. The green wavelength component of the wavelength thus transmitted is incident on the light amplifying tube 35, but due to the gate function of the light amplifying tube 35 as illustrated in chart 104, only the valid component transmitted by the G region 33c is amplified by the light amplifying tube 35. The light of the green wavelength component amplified by the light amplifying tube 35 is captured by the CCD camera 37, which accumulates an electric charge corresponding to an image signal. The image signal of the red wavelength component output to the RGB frame memory 42 is written to the Red VFM of the RGB frame memory 42, as shown in chart 108. The characteristic noise components generated by amplification of the light in the light amplifying tube 35 are removed from the image signal of the red wavelength component written to the Red VFM, by means of the image quality enhancing device 43. Thereupon, the image signal is input to the computing section of the scan converter 44, as shown in chart 113, and superimposed with the previously stored image signal of the extended red wavelength component.

At time point t₄, the CCD charge read-out signal rises, as shown in chart 109, whereby the electric charge corresponding to the image signal of the green wavelength component accumulated in the CCD camera 37 is output to the RGB frame memory 42 of the RGB control unit 3. In the time period between time point t₄ and t₅, the weak light is transmitted by the B region 33d of the rotating RGB filter 33, as illustrated by chart 103. The blue wavelength component of the weak light thus transmitted is incident on the light amplifying tube 35, but due to the gate function of the light amplifying tube 35 as illustrated in chart 104, only the valid component transmitted by the B region 33d is amplified by the light amplifying tube 35. The light of the blue wavelength component amplified by the light amplifying tube 35 is captured by the CCD camera 37, which accumulates an electric charge corresponding to the image signal. The image signal of the green wavelength component that was output to the RGB frame memory 42 is written to the Green VFM of the RGB frame memory 42, as shown in chart 110. The characteristic noise components generated by amplification of the light in the light amplifying tube 35 are removed from the image signal of the green wavelength component written to the Green VFM, by means of the image quality enhancing device 43. Thereupon, the image signal is input to the computing section of the scan converter 44, as shown in chart 113, and superimposed with the previously input image signals of the extended red, and red wavelength components.

At this time point, the RGB filter has made one revolution, and images for all wavelength components have been captured by the CCD camera 37. As described hereinafter, it is possible to obtain a single color image by reading out all of the captured image signals. In this way, since one color image is created for every 4 clocks of the video signal VD shown in chart 102, a color image which changes at a frequency of 15 Hz is obtained. Thereafter, the operations in charts 101 to 104 illustrated in Fig. 7A to Fig. 7D are repeated from time point t₁, and the corresponding operations of reading out the CCD charge, and the like, are also repeated, further description thereof being omitted here.

At time point t₅, the CCD charge read-out signal rises as shown in chart 111, whereby the electric charge corresponding to the blue wavelength component accumulated in the CCD camera 37 is output to the RGB frame memory 42 of the RGB control unit 3. In the time period from time point t₅ to t₆, the image signal of the blue wavelength component output to the RGB frame memory 42 is written to the Blue VFM of the RGB frame memory 42, as shown in chart 112. The characteristic noise components generated by amplification of the light in the light amplifying tube 35 are removed from the image signal of the blue wavelength component written to the Blue VFM, by means of the image quality enhancing device 43. Thereupon, the image signal is input to the computing section of the scan converter 44, as shown in chart 113, where it is superimposed with the previously input image signals of the extended red, red, and green wavelength components. Here, an image signal wherein the image signals of the red, red, green and blue wavelength components (Ext. R + R + G + B) are superimposed is stored in the computing section of the scan converter 44.

At time point t₆, the (Ext. R + R + G + B) read-out signal rises, as shown in chart 114, and the (Ext. R + R + G + B) image signal stored in the computing section of the scan converter 44 is read out. Here, the scan converter 44 converts the scanning of the (Ext. R + R + G + B) image signal in accordance with the connected media to which the image are to be input. For example, the image signal to be written to the monitor 27 is converted to an NTSC signal. In this case, the scan converter 44 converts the (Ext. R + R + G + B) image signal to an image signal for Odd or Even interlace scanning, as illustrated in chart 115 in Fig. 70, in order to comply with an NTSC image signal. In this first embodiment, as well as a monitor 27, the media to which the images are input also comprise a VTR 28 and PC 29, as illustrated in Fig. 1. The scan converter 44 converts the (Ext. R + R + G + B) image signal to an NTSC signal for input to the monitor, to a Y/C signal for input to the VTR 28, and to an RGB signal for input to the PC. Here, the PC 29 inputs the converted RGB signal by means of an interface circuit (I/F) 29a.

The input image is display by the monitor 27, and the like, whereby the observer is able to view a color image of the subject A. Incidentally, the weak light color imaging device of the first embodiment captures images of the red (or infrared) wavelength components at a higher ratio than images as seen by the human eye, and therefore, the images obtained have a strong red intensity.

In the first embodiment, when an excitation light source 22 is irradiated onto a subject A comprising cancer cells, HpD fluorescence having peaks at word lines of 630 nm and 690 nm are generated by the subject A. Here, a case is described where a subject A is imaged by means of a weak light color imaging device according to the first embodiment. Incidentally, both the variable-output white light source 21 and excitation light source 22 are irradiated onto the subject A and the status of the peripheral region of the subject A is observed simultaneously with the status of fluorescence generation.

The action of the first embodiment is described in a case where an excitation light source 22 is used. The excitation light component contained in the weak light from the subject A guided to the camera box 3 by the endoscope 11 is cut out by the excitation light excluding filter 31. The weak light from which this excitation light component has been removed passes through the coupling lens 32 and is then incident on, and filtered by, the RGB filter 33. The action thereafter is similarly to the action of the first embodiment described above, and therefore further description thereof is omitted here.

The fluorescence of 630 nm and 690 nm wavelength from subject A, as captured by the weak light color imaging device according to the first embodiment, is contained in the extended red wavelength component and red wavelength component, and hence it is emphasized in the captured images. Consequently, both the image of the peripheral region of the subject A and the fluorescence of the HpD are readily discernable by the observer on the monitor 27 displaying the captured images, and therefore he or she can perform PDD easily. After establishing the location of cancer cells by means of PDD, the excitation light source 22 can be changed for a treatment light source 23, whereby PDT can be performed at that location.

If the fluorescence of the HpD is extremely weak, or if the intensity of the illumination light is strong, then the fluorescence may become swamped by the illumination light for the subject A as irradiated by the white light source 21, and hence it may not be possible to distinguish the fluorescence from the captured images displayed on the monitor 27. Therefore, whilst observing the image of subject A displayed on the monitor 27, the output of the white light source 21 is adjusted appropriately such that the intensity of the light from the white light source 21 that is incident on the endoscope 11 due to reflection or scattering by subject A becomes equal to, or equal to or lower than, the intensity of the fluorescence that is incident on the endoscope 11 from the subject A, in such a manner that the fluorescence can be distinguished whilst still being able to observe the appearance of the peripheral regions of the subject A. Thereby, the observer is able to identify the location at which fluorescence is being generated in the subject A, and hence he or she can perform PDD readily.

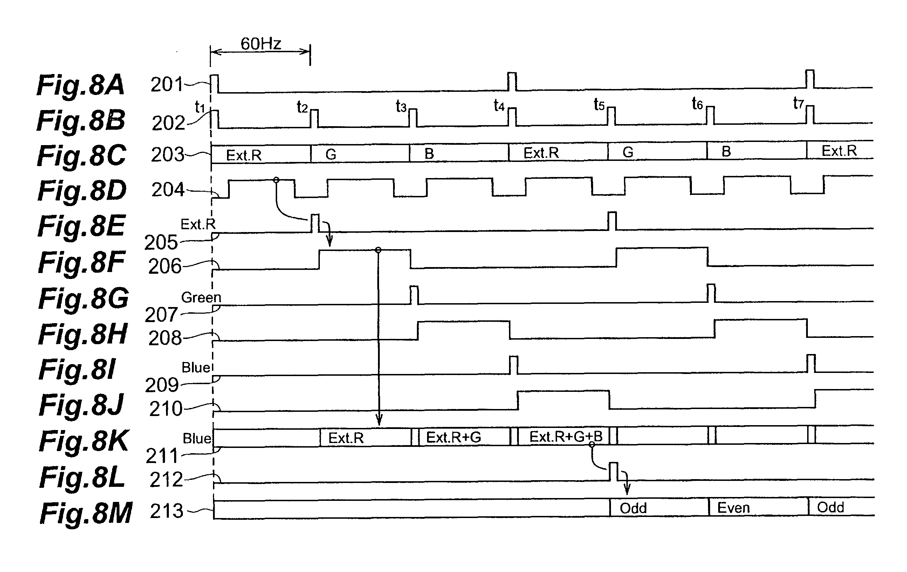

Next, the action of the first embodiment is described in a case where the filter in Fig. 5B is used as the RGB filter 33, with reference to Fig. 8A - Fig. 8M. Fig. 8A - Fig. 8M are timing charts indicating the operations performed for each signal from inputting images of the various wavelength components, extended red, green and blue, in synchronism with a 60 Hz VD signal, through computation of the respective images, until generation of a single color image. In the diagram, chart 201 shows the start signal, chart 202 shows the video signal (VD), chart 203 shows the RGB filter signal, chart 204 shows the gate signal of the light amplifying tube 35, chart 205 shows the Ext. R charge read-out signal of the CCD, chart 206 shows the Ext. R VFM input signal, chart 207 shows the CCD Green charge read-out signal, chart 208 shows the Green VFM input signal, chart 209 shows the CCD Blue charge read-out signal, chart 210 shows the Blue VFM input signal, chart 211 shows the (Ext. R + G + B) computing signal, chart 212 shows the (Ext. R + G + B) read-out signal, and chart 213 shows the interlace scan converting signal.

The operations in charts 201 to 213 shown in Fig. 8A to Fig. 8M are virtually the same as the actions in the first embodiment described above, and detailed description thereof is omitted, but the point of difference with respect to Fig. 7A to Fig. 70 is that the RGB filter 33 is divided into three, and therefore the images of the respective extended red, green and blue wavelength components are input in 3 clocks of the VD signal in chart 202 shown in Fig. 8B, and hence one color image is obtained every 3 clocks. In other words, using the RGB filter 33 shown in Fig. 5B, a color image which changes at a frequency of 20 Hz is obtained. As described above, with the RGB filter 33 shown in Fig. 5A, a color image of 15 Hz is obtained, so it can be seen that better image quality can be obtained by using the RGB filter 33 shown in Fig. 5B. However, when using the RGB filter 33 in Fig. 5A, the extended red and red wavelength components are imaged for a longer period of time, and therefore a color image having a greater red wavelength component can be obtained.

In this way, the weak light color imaging device according to the first embodiment is able to capture wavelength containing HpD fluorescence components from a subject A, in the form of moving, color images, and color images in which both the appearance of the peripheral region of the subject A and the fluorescence of the HpD can be observed are obtainable by means of images in which the fluorescence of the HpD contained in the subject A is emphasized.

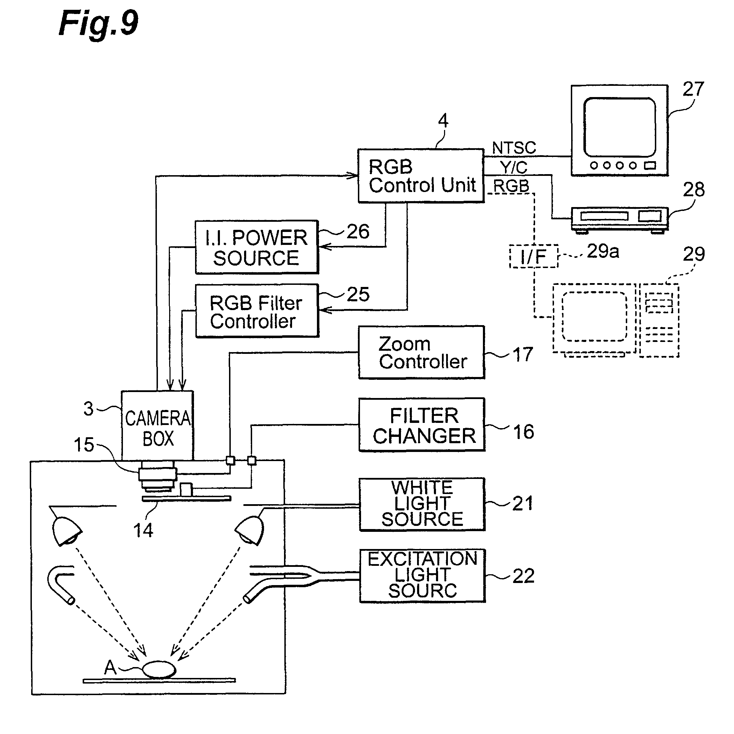

Next, a second embodiment of the present invention is described with reference to Fig. 9.

Fig. 9 is a diagram showing an approximate diagram of a second embodiment. In this second embodiment, a weak light color imaging device is lens mounted and used for macro imaging, for example, in cases where the subject A is placed directly in the line of sight, during surgery, or the like, and images of the subject A are captured directly. Similarly to the first embodiment, it is assumed that HpD has already been injected into the patient's body. As shown in Fig. 9, the weak light color imaging device according to this second embodiment comprises: two types of light source 21 and 22, a zoom lens 15 for magnifying the subject A at a prescribed rate of magnification, a camera box 3 for amplifying and capturing images of the weak light from the subject A, for the respective red, green and blue wavelength components, an RGB control unit 4 for obtaining a single image by computing the captured images signals for the respective wavelength components, a monitor 27, VTR 28 and PC 29 for displaying and storing the images thus obtained, and other peripheral devices.

To give a more detailed description, the weak light color imaging device according to this second embodiment comprises: a white light source 21 which irradiates illumination light onto a subject A comprising cancer cells, and an excitation light source 22 which irradiates excitation light onto the subject A, thereby exciting the HpD accumulated in the subject A and causing it to generate fluorescence. In contrast to the first embodiment, the subject A is placed directly in the line of sight, and hence the illumination light from the white light source 21 can be irradiated onto the subject A directly, without passing via a light guide, or the like. Moreover, the excitation light source 22 is guided along a fibre in order to be irradiated onto the subject A.

The weak light color imaging device according to the second embodiment comprises: an excitation light excluding filter 14 for excluding excitation light for exciting the HpD that is reflected or scattered by the subject A, a zoom lens 15 for magnifying the weak light from the subject A by a prescribed magnification factor, a filter converter 16 for performing control for changing the filter used for the excitation light excluding filter to a filter corresponding to the wavelength of the excitation light used, by rotating the excitation light excluding filter 14, or conducting the weak light from the subject A directly onto the zoom lens 15, without passing the filter, in cases where the excitation light is not irradiated, and a zoom controller 17 for controlling the magnification factor of the zoom lens 15. In the first embodiment, the excitation light excluding filter 14 was provided in the camera box 3, as shown in Fig. 1, but in this second embodiment, it is provided externally, rather than inside a camera box 3.

Moreover, the weak light color imaging device according to the second embodiment comprises a camera box 3 for imaging the weak light from the subject A, and an RGB control unit 4 for storing the respective image signals of the extended red, red, green and blue wavelength components of the wavelength captured by the camera box 3, and converting same to image signals corresponding to the media to which the images are to be input. The internal composition of the camera box 3 and the RGB control unit 4 is the same as that of the first embodiment of the weak light color imaging device illustrated in Fig. 1. However, as stated previously, in the second embodiment, the excitation light excluding filter 31 provided inside the camera box 3 in the foregoing description is positioned between the subject A and the camera box 3, rather than inside the camera box 3.

Moreover, as in the first embodiment, an RGB filter controller 25 for controlling the rotation of the RGB filter 33 inside the camera box 3, in synchronism with a start signal sent by a CCU 41 provided inside the RGB control unit 4, and a light amplifying tube power source 26 for controlling a gate function of the light amplifying tube 35 inside the camera box 3 are also provided. A monitor 27, VTR 28 and PC 29 are provided as media to which the captured image signals are input. Here, the PC 29 displays images by inputting an RGB signal of which the scanning has been converted by the RGB control unit 4, by means of an interface circuit 29a.

Next, with reference to Fig. 9, the action of the weak light color imaging device according to the second embodiment is described. Firstly, the white light source 21 and the excitation light source 22 are irradiated onto the subject A. The weak light containing HpD fluorescence from the subject A is incident on the zoom lens 15 via the excitation light excluding filter 14. The zoom lens 15 magnifies the image of the subject A based on the weak light, by a prescribed factor of magnification, and inputs this image to the camera box 3. The action of the camera box 3 and subsequent elements is the same as the first embodiment and is omitted here.

In this way, in the second embodiment, the weak light color imaging device according to the present invention is lens mounted, and can be used to capture macro images of a subject A positioned directly in the line of sight. The weak light containing an HpD fluorescence component from the subject A can be captured in the form of moving color images, and color images are obtainable wherein both the appearance of the peripheral region of the subject A and the fluorescence of the HpD can be observed, by means of images wherein the fluorescence of the HpD contained in the subject A is emphasized.

The present invention is not limited to the first or second embodiments. For example, in the first embodiment and second embodiment, in order to capture images on the CCD camera 37 of only the valid components of the weak light transmitted by the RGB filter 33, a gate function of the light amplifying tube 35 is employed, but it is also possible to capture only the valid component of the amplified weak light by using a shutter function of the CCD camera 37. Moreover, here, the VTR 28 was connected to the scan converter 44, but it is also possible to input (record) the images displayed on the monitor 27 thereto, by connecting same to the monitor 27. Moreover, in the first and second embodiments, since an image of the infrared wavelength component is also captured, it is possible to perform PDD in combination with photosensitive materials other than HpD, which generate fluorescence in the infrared wavelength region. Furthermore, it is also possible to conceive of other application examples, where, similarly to the second embodiment, the weak light color imaging device according to the present invention is lens mounted for use in a microscope, and microscopic image capturing is performed.

### INDUSTRIAL APPLICABILITY

As described in detail above, by means of the weak light color imaging device according to the present invention, the wavelength from a subject A is transmitted by an RGB filter, and amplified by a light amplifying tube, whereby moving, color images of the subject can be obtained.

Moreover, by adjusting the output of the white light source irradiated illumination light, in such a manner that that the weak fluorescence from the HpD is not swamped by the illumination light irradiated onto the subject, it is possible to capture images of both the fluorescence of the HpD and the peripheral region of the subject. Therefore, it is possible to identify the location of HpD fluorescence in the subject, and hence PDD can be performed readily.

Moreover, by using a filter with enhanced transmissivity of longer wavelengths, especially, red (and infrared) wavelength components, for the RGB filter, or by increasing the surface area of the filter that transmits light of red (and infrared) wavelength components above that of the filter transmitting light of green and blue wavelength components, or by transmitting light of red (and infrared) wavelength components for a longer period of time, it is possible to obtain images of the subject containing a large quantity of red wavelength component. Thereby, since the fluorescence of the HpD contained in the subject is emphasized in the captured images, it is possible to perform PDD visually from the captured images.

Moreover, since images of the infrared wavelength component can also be captured, it is possible to perform PDD using photosensitive materials other than HpD, which generate fluorescence in the infrared wavelength region when irradiated with excitation light.

## Claims

1. A weak light color imaging device for capturing color images of the weak light from a subject (A), comprising:
illuminating means (21) for illuminating said subject (A);
exciting means (22) for causing fluorescence to be generated by irradiating excitation light onto said subject (A);
filtering means (33) for respectively transmitting the red, green and blue wavelength components of the weak light from said subject (A);
light amplifying means (35) for amplifying the light intensity of the respective red, green and blue wavelength components of the weak light from said subject (A) as transmitted by said filtering means (33), and for outputting the amplified light;
imaging means (37) for capturing images of the respective red, green and blue wavelength components of the amplified light from said subject (A);
storing means (42) for storing image signals of the respective red, green and blue wavelength components thus captured;
converting means (44) for superimposing the image signals for said red, green and blue wavelength components stored by said storing means, and converting same to a color image signal;
monitoring means (27) for displaying said color image signal as a color image; and
output varying means for varying the output of said illuminating means.

2. The weak light color imaging device according to claim 1, **characterized in that** said output varying means varies the output of said illuminating means (21) in such a manner that the intensity of the light from said illuminating means (21) incident on said imaging means (37)due to reflection or scattering by said subject is equal to or less than the light intensity of the fluorescence incident on said imaging means (37) from said subject (A).

3. The weak light color imaging device according to claim 1, **characterized in that** said filtering means (33) transmits a greater quantity of said red wavelength component of the weak light from said subject (A) than said blue and green wavelength components.

4. The weak light color imaging device according to claim 3, **characterized in that** said filtering means (33) transmits a greater quantity of said red wavelength component of the weak light from said subject than said blue and green wavelength components, by transmitting said red wavelength component for a longer period of time than said blue and green wavelength components.

5. The weak light color imaging device according to claim 3, **characterized in that** said filtering means (33) transmits a greater quantity of said red wavelength component of the weak light from said subject than said blue and green wavelength components, by transmitting said red wavelength component at a higher transmissivity than said green and blue wavelength components.

6. The weak light color imaging device according to claim 1, **characterized in that** said filtering means (33) also transmits a wavelength component in the infrared region.

## Patentansprüche

1. Farbbildaufnahmegerät für schwaches Licht zum Aufnehmen von Farbbildern des schwachen Lichts von einem Gegenstand (A), das Folgendes umfasst:
ein Beleuchtungsmittel (21) zum Beleuchten des Gegenstandes (A),
ein Erregungsmittel (22), das durch Bestrahlen des Gegenstandes (A) mit Erregungslicht die Erzeugung von Fluoreszenz verursacht,
ein Filtermittel (33), das jeweils den roten, den grünen und den blauen Wellenlängenbestandteil des schwachen Lichts von dem Gegenstand (A) durchlässt,
ein Lichtverstärkungsmittel (35) zum Verstärken der Lichtintensität des jeweiligen roten, grünen und blauen Wellenlängenbestandteils des schwachen Lichts von dem Gegenstand (A), wie sie von dem Filtermittel (33) durchgelassen werden, und zum Ausgeben des verstärkten Lichts,
ein Bildaufnahmemittel (37) zum Aufnehmen von Bildern des jeweiligen roten, grünen und blauen Wellenlängenbestandteils des verstärkten Lichts von dem Gegenstand (A),
ein Speichermittel (42) zum Speichern von Bildsignalen des jeweiligen so aufgenommenen roten, grünen und blauen Wellenlängenbestandteils,
ein Umwandlungsmittel (44) zum Überlagern der Bildsignale des von dem Speichermittel gespeicherten roten, grünen und blauen Wellenlängenbestandteils und Umwandeln dieser in ein Farbbildsignal,
ein Überwachungsmittel (27) zum Anzeigen des Farbbildsignals als Farbbild, und
ein Ausgabeveränderungsmittel zum Variieren der Ausgabe des Beleuchtungsmittels.

2. Farbbildaufnahmegerät für schwaches Licht nach Anspruch 1, **dadurch gekennzeichnet, dass** das Ausgabeveränderungsmittel die Ausgabe des Beleuchtungsmittels (21) auf eine solche Weise variiert, dass die Intensität des Lichts vom Beleuchtungsmittel (21), das auf das Bildaufnahmemittel (37) einfällt, aufgrund von Reflexion oder Streuung durch den Gegenstand gleich der oder geringer als die Lichtintensität der vom Gegenstand (A) auf das Bildaufnahmemittel (37) einfallenden Fluoreszenz ist.

3. Farbbildaufnahmegerät für schwaches Licht nach Anspruch 1, **dadurch gekennzeichnet, dass** das Filtermittel (33) eine größere Menge des roten Wellenlängenbestandteils des schwachen Lichts von dem Gegenstand (A) durchlässt als vom blauen und vom grünen Wellenlängenbestandteil.

4. Farbbildaufnahmegerät für schwaches Licht nach Anspruch 3, **dadurch gekennzeichnet, dass** das Filtermittel (33) eine größere Menge des roten Wellenlängenbestandteils des schwachen Lichts von dem Gegenstand durchlässt als vom blauen und vom grünen Wellenlängenbestandteil, indem es den roten Wellenlängenbestandteil für eine längere Zeitdauer durchlässt als den blauen und den grünen Wellenlängenbestandteil.

5. Farbbildaufnahmegerät für schwaches Licht nach Anspruch 3, **dadurch gekennzeichnet, dass** das Filtermittel (33) eine größere Menge des roten Wellenlängenbestandteils des schwachen Lichts von dem Gegenstand durchlässt als vom blauen und vom grünen Wellenlängenbestandteil, indem es den roten Wellenlängenbestandteil mit einem höheren Durchlässigkeitsgrad durchlässt als den blauen und den grünen Wellenlängenbestandteil.

6. Farbbildaufnahmegerät für schwaches Licht nach Anspruch 1, **dadurch gekennzeichnet, dass** das Filtermittel (33) auch einen Wellenlängenbestandteil im Infrarotbereich durchlässt.

## Revendications

1. Dispositif d'imagerie en couleurs à faible lumière pour prendre des images en couleurs d'un sujet (A) en lumière faible, comprenant :
un moyen d'éclairage (21) pour éclairer ledit sujet (A) ;
un moyen d'excitation (22) pour provoquer la génération d'une fluorescence en irradiant ledit sujet (A) d'une lumière d'excitation ;
un moyen de filtrage (33) pour transmettre respectivement les composantes de longueur d'onde rouge, verte et bleue de la lumière faible provenant dudit sujet (A) ;
un moyen d'amplification de lumière (35) pour amplifier l'intensité lumineuse des composantes de longueur d'onde respectives rouge, verte et bleue de la lumière faible provenant dudit sujet (A) transmises par ledit moyen de filtrage (33), et pour délivrer en sortie la lumière amplifiée ;
un moyen d'imagerie (37) pour prendre des images des composantes de longueur d'onde respectives rouge, verte et bleue de la lumière amplifiée provenant dudit sujet (A) ;
un moyen de stockage (42) pour stocker des signaux d'image des composantes de longueur d'onde respectives rouge, verte et bleue ainsi capturées ;
un moyen de conversion (44) pour superposer les signaux d'image pour lesdites composantes de longueur d'onde rouge, verte et bleue stockées par ledit moyen de stockage, et pour les convertir en un signal d'image en couleurs ;
un moyen de surveillance (27) pour afficher ledit signal d'image en couleurs sous la forme d'une image en couleurs ; et
un moyen de variation de sortie pour faire varier la sortie dudit moyen d'éclairage.

2. Dispositif d'imagerie en couleurs à faible lumière selon la revendication 1, **caractérisé en ce que** ledit moyen de variation de sortie fait varier la sortie dudit moyen d'éclairage (21) de manière telle que l'intensité de la lumière provenant dudit moyen d'éclairage (21) et arrivant sur ledit moyen d'imagerie (37), due à la réflexion ou à la dispersion réalisée par ledit sujet est inférieure ou égale à l'intensité lumineuse de la fluorescence arrivant sur ledit moyen d'imagerie (37) depuis ledit sujet (A).

3. Dispositif d'imagerie en couleurs à faible lumière selon la revendication 1, **caractérisé en ce que** ledit moyen de filtrage (33) transmet une plus grande quantité de ladite composante de longueur d'onde rouge de la lumière faible provenant dudit sujet (A) que desdites composantes de longueur d'onde bleue et verte.

4. Dispositif d'imagerie en couleurs à faible lumière selon la revendication 3, **caractérisé en ce que** ledit moyen de filtrage (33) transmet une plus grande quantité de ladite composante de longueur d'onde rouge de la lumière faible provenant dudit sujet que desdites composantes de longueur d'onde bleue et verte, en transmettant ladite composante de longueur d'onde rouge pendant un intervalle de temps plus long que lesdites composantes de longueur d'onde bleue et verte.

5. Dispositif d'imagerie en couleurs à faible lumière selon la revendication 3, **caractérisé en ce que** ledit moyen de filtrage (33) transmet une plus grande quantité de ladite composante de longueur d'onde rouge de la lumière faible provenant dudit sujet que desdites composantes de longueur d'onde bleue et verte, en transmettant ladite composante de longueur d'onde rouge avec un coefficient de transmission plus élevé que lesdites composantes de longueur d'onde bleue et verte.

6. Dispositif d'imagerie en couleurs à faible lumière selon la revendication 1, **caractérisé en ce que** ledit moyen de filtrage (33) transmet aussi une composante de longueur d'onde du domaine infrarouge.
